# EUROPEAN PATENT SPECIFICATION

(11) **EP 1 869 985 B1**
(45) Date of publication and mention of the grant of the patent: **23.05.2018**
(21) Application number: 05728721.1
(22) Date of filing: 08.04.2005
(51) Int. Cl.: A23K 20/147, A23L 33/165, A23L 33/19, A23C 21/10, A23C 9/152, A23K 20/20

(54) **USE OF A MILK PROTEIN IN A COMPOSITION COMPRISING IRON AND VITAMIN C TO AVOID AN ASTRINGENT TASTE OF IRON OR THE FORMATION OF PRECIPITATES, WHEREIN THE MILK PROTEIN IS SKIM MILK OR WHEY PROTEIN ISOLATE**
VERWENDUNG EINES MILCHPROTEINS IN EINER ZUSAMMENSETZUNG, DIE EISEN UND VITAMIN C ENTHÄLT, UM EINEN ADSTRINGIERENDEN GESCHMACK VON EISEN ODER DIE BILDUNG VON SINKSTOFFEN ZU VERMEIDEN, WOBEI DAS MILCH PROTEIN MAGERMILCH ODER MOLKEPROTEINISOLAT IST
UTILISATION D'UNE PROTÉINE DE LAIT DANS UNE COMPOSITION COMPRENANT DU FER ET DE LA VITAMINE C AFIN D'ÉVITER UN GOÛT ASTRINGENT DE FER OU LA FORMATION DE PRÉCIPITÉS, LA PROTÉINE DE LAIT ÉTANT DU LAIT ÉCRÉMÉ OU UN ISOLAT DE PROTÉINE DE LACTOSÉRUM

(43) Date of publication of application: 26.12.2007
(73) Proprietor: Megmilk Snow Brand Co., Ltd., Sapporo-shi Hokkaido 0650043 (JP)
(72) Inventor: Ueda, Noriko c/o Snow Brand Milk Products Co., Ltd, Kawagoe-shi, Saitama; 3501165 (JP); Kato, Ken c/o Snow Brand Milk Products Co., Ltd., Kawagoe-shi, Saitama; 3501165 (JP); Tanaka, Miyako Snow Brand Milk Products Co., Ltd., Kawagoe-shi, Saitama; 3501165 (JP); Kakehi, Yuji c/o Snow Brand Milk Products Co., Ltd, amidai, Kawagoe-shi, Saitama; 3501165 (JP); Sato, Kaoru c/o Snow Brand Milk Products Co., Ltd., Kawagoe-shi, Saitama; 3501165 (JP); Yoshioka, T. c/o Snow Brand Milk Products Co.,Ltd., Kawagoe-shi, Saitama; 3501165 (JP)
(74) Representative: Wakerley, Helen Rachael
(86) International application number: PCT/JP2005/006899
(87) International publication number: WO 2006/114840

(56) References cited:
- WO-A1-98/43495
- JP-A- 4 141 067
- JP-A- 5 155 774
- JP-A- 10 191 933
- JP-A- 2002 000 225
- JP-A- 2004 236 589
- JP-A- 2005 110 636
- JP-A- 2005 119 983
- KR-A- 20040 048 749
- US-A- 5 002 779
- SOUCI-FACHMANN-KRAUT: "Food composition and nutrition tables" 2000, MEDPHARM SCIENTIFIC PUBLISHERS , STUTTAGART , XP002523638 ISBN: 978-3-8047-5083-8 * pages 6,7 * * page 51 - page 56 * * pages 133,134 *
- DATABASE WPI Week 198812 Thomson Scientific, London, GB; AN 1988-082497 XP002523639 & SU 1 329 743 A (KIEV PEDIATRICS) 15 August 1987 (1987-08-15)

## Description

### Technical Field

The invention is defined as set forth in the claims. All other embodiments are described for illustrative purposes only, and any statement that they would also be part of the invention or other, similar statements, have to be seen in the context of the application as filed and do not alter the scope of the claims.

### Background Art

It is known that among minor elements, iron that is taken as a nutrient is insufficient around the world, and there are 4.0 to 5.0 billion of iron-deficiency anemia patients including potential patients throughout the world. Intake of iron is insufficient in Japan, particularly in young women (see Non-Patent Document 1, for example). Therefore, iron is one of the nutrients to be sufficiently taken in daily eating. However, absorbability of iron varies depending on the form of a food containing iron, and intake of iron from daily meals alone may be insufficient. Therefore, it is encouraged to use iron-enriched foods or supplements or to take iron together with a substance that is said to enhance the absorbability of iron, such as vitamin C (ascorbic acid) (see Non-Patent Document 2, for example) . Forthisreason, inrecentyears, manyironmaterials and iron-enriched foods have been developed.

Examples of the developed iron materials include heme iron, iron lactate, iron citrate, a bicarbonic acid-iron-casein complex (see Patent Document 1, for example), dried iron casein powder (see Patent Document 2, for example), an iron-whey protein hydrolysate complex (see Patent Document 3, for example), and an iron-lactoferrin complex (see Patent Document 4, for example), and production technologies for the materials have been disclosed. In the present invention, among the iron materials, materials that are generally used in foods or drinks , animal feeds, and medicines are referred to as iron preparations.

However, those are products developed as iron materials with high iron contents and cannot prevent iron release or reduction caused by a substance having a property of releasing iron or reducing the released iron. That is, if a substance capable of enhancing the absorbability of iron (such as vitamin C) is blended into the iron materials, iron is released or further reduced, thereby causing astringent taste of iron or forming precipitates.

In order to prevent such phenomena, a coated vitamin C preparation may be used, but in the case where the preparation is mixed and heat-sterilized in the form of a liquid, it is impossible to completely suppress the astringent taste of iron or formation of precipitates. Therefore, at present, it is difficult to mix a substance capable of enhancing the absorbability of iron (such as vitamin C) in iron-enriched foods and drinks.
Patent Document 1: JP Hei 09-77793 A
Patent Document 2: JP Hei 11-75707 A
Patent Document 3: JP 2000-50812 A
Patent Document 4: JP Hei 07-304798 A
Non-Patent Document 1: Status of National Nutrition (the results of the national nutrition survey in 2001), edited by the Society of Health/Nutrition Information, June 10, 2003, Dai-ichi Shuppan Publishing Co., Ltd.
Non-patent Document 2: J.N.Counsell and D.H.Hornig, VITAMIN C, Applied Science Publishers, 49-61, 1981 Patent Document US5002779 relates to nutritional improvements in vitamin C and iron supplemented chocolate powders or flavoured beverage mixes which preferably contain non-fat milk solids.

### Disclosure of the Invention

### Problems to be solved by the Invention

The present invention is as set forth in the claims.

### Means for solving the Problems

The inventors of the present invention have made intensive studies on stabilization of iron for solving the above-described problems, and as a result, they found out that an iron composition obtained by blending a milk protein such as skim milk into an iron preparation is stable even in the presence of a substance having a property of releasing iron or reducing the released iron, such as vitamin C and citric acid. The present invention has been completed based on the above findings.

Therefore, the present invention relates to an iron composition including a milk protein and an iron preparation, which is stable in the presence of a substance having a property of releasing iron or reducing the released iron.

In addition, the present invention relates to an iron composition characterized by being stable in the presence of a substance, which is obtained by mixing a rawmaterial that is generally used in foods or drinks , animal feeds, and medicines into a milk protein and an iron preparation, and has a property of releasing iron or reducing the released iron.

Further, the present invention relates to foods or drinks, animal feeds, and medicines blended with an iron composition including a milk protein and an iron preparation, which is characterized by being stable in the presence of a substance having a property of releasing iron or reducing the released iron.

### Effect of the Invention

According to the present invention, it is possible to provide a stable iron composition in the presence of a substance having a property of releasing iron from an iron preparation or reducing the released iron, without causing an astringent taste of iron and forming precipitates or the like.

### Best Mode for carrying out the Invention

A feature of the present invention is tomixa milk protein such as skim milk into an iron preparation.

Examples of the milk protein include milk protein-containing products such as raw milk, skim milk, whey, and desalted whey, and any one of concentrates or dried products thereof, a whey protein concentrate (WPC), a whey protein isolate (WPI), and casein may be used as long as containing the milk protein. Examples of the iron preparation include: inorganic iron preparations such as ferrous sulfate and ferric pyrophosphate; and organic iron preparations such as heme iron, ferritin iron, and lactoferrin iron. An iron composition containing a milk protein of the present invention may be prepared by mixing those milk proteins into an iron preparation or by mixing those milk proteins into a raw material with a relatively high iron content. In addition, an iron composition containing a milk protein of the present invention may be prepared by mixing a raw material that is generally contained in other foods or drinks, animal feeds, and medicines (such as sugar, lipid, or flavor) into an iron composition. Moreover, it is possible to provide foods or drinks, animal feeds, and medicines blended with an iron composition blended with a milk protein of the present invention.

With regard to a ratio between a milk protein content and an iron preparation content in an iron composition of the present invention, the composition preferably contains 15 parts or more of a milk protein to 1 part of iron in an iron preparation.

In the case where an iron composition is prepared by mixing a milk protein into a raw material or the like with a relatively high iron content, the protein and material are blended at the above-described content ratio based on a calculation of an iron content in the raw material. If a milk protein is contained in the amount of less than 15 parts with respect to 1 part of iron in the iron preparation, the composition cannot be stable in the presence of a substance having a property of releasing iron or reducing the released iron to cause an astringent taste of iron. That is, the inventors of the present invention have found out that, if a milk protein is contained in an amount of 15 parts or more, the milk protein content to the iron content increases, resulting in a higher stability of the iron composition in the presence of a substance having a property of releasing iron or reducing the released iron to cause astringent taste of iron.

A method of preparing an iron composition of the present invention obtained by mixing a milk protein and an iron preparation is not particularly limited, and for example, for preparing the composition in a solution, the method includes the following steps: suspending or dissolving a milk protein and an iron preparation in deionized water; stirring and mixing the solution; and producing a food or drinks, animal feed, or medicine from the mixture. The conditions of stirring and mixing are not particularly limited as long as a milk protein and an iron preparation can be mixed sufficiently, and it is possible to perform the stirring and mixing using an ultra disperser while heating at about 30 to 40°C, if necessary. A solution of the iron composition may be optionally concentrated using an RO membrane or dried by spray-drying, freeze-drying, or the like so that the solution can be easily used in foods or drinks, animal feeds, and medicines.

An iron composition of the present invention is stable in the presence of a substance having a property of releasing iron from an iron preparation or reducing the released iron to cause an astringent taste of iron (such as vitamin C and citric acid), and the composition can be subjected to sterilization treatment that is generally used in production of foods or drinks, animal feeds, and medicines. Even if the composition is powder, it can be subjected to dry-heat sterilization. Therefore, the composition can be used for preparing foods or drinks, animal feeds, and medicines in various forms such as liquid, gel, powder, and granule.

The astringent taste of iron is said to be caused by reducing iron from trivalent to divalent by a substance having a property of releasing iron or reducing the released iron. If iron is present together with a compound having C-COOH and C-OH as partial structures (such as citric acid), iron may be released from an iron preparation to form coordinate bonds or become unstable, resulting in formation of precipitates. Examples of such a substance having a property of releasing iron or reducing the released iron to cause an astringent taste of iron or formation of precipitates include, but not limited to, vitamin C, sodium ascorbate, sugar (saccharide having a reducing terminal), and citric acid.

An iron composition of the present invention may be an iron composition containing only a milk protein and an iron preparation, or an iron composition containing not only a milk protein and an iron preparation but also another raw material or the like generally contained in foods or drinks, animal feeds, and medicines (such as sugar or flavor) . In addition, it is possible to provide: foods or drinks, animal feeds, and medicines including an iron composition containing only a milk protein and an iron preparation; or foods or drinks, animal feeds, and medicines including an iron composition containing a raw material generally contained in foods or drinks, animal feeds, and medicines.

Hereinafter, the present invention will be described in detail by way of Test Examples and Examples, which merely show embodiments of the present invention as examples, and the present invention is not limited by these Examples.

### Example 1

An iron-saturated lactoferrin solution with an iron content of 1% (hereinafter, referred to as iron LF solution, 6.2 kg) and skim milk (130 kg) were mixed and subjected to homogenization treatment using a homogenizer at 150 kg/cm², followed by UHT sterilization using a UHT plate heat sterilizer. The resultant solution was concentrated by heating at 80°C and then spray-dried, to thereby yield 9.2 kg of an iron composition containing skim milk.

### Test Example 1

The iron composition obtained in Example 1 (40 g) was dissolved in sterilized deionized water (160 g) to prepare 200 g of an aqueous solution. To the aqueous solution was added 200 mg of vitamin C (L-ascorbic acid, manufactured by Tanabe Seiyaku Co. , Ltd.), followed by observation of formation state of precipitates, sensory evaluation, and measurement of free iron.

The sensory evaluation was performed by ten panelists specialized in sensory evaluation. The astringent taste of each sample was evaluated in the following four levels, and the average of the points was calculated.
0 point: no astringent taste
1 point: weak astringent taste
2 points: rather strong astringent taste
3 points: strong astringent taste

The free iron was measured by: performing ultrafiltration of each sample using an ultrafilter unit USY-1 (molecular weight cut-off: 10,000) ; and measuring absorbance at 750 nm using FeC-test Wako (manufactured by Wako Pure Chemical Industries, Ltd.) to determine the iron content in the filtrate.

The test results are shown in Table 1. As is clear from Table1, the solution obtained by adding vitaminC to an iron composition solution containing skim milk was found to have no astringent taste, contain little free iron, and formno precipitates despite the blending of a high level of iron (20 mg%) . On the other hand, the solution obtained by adding vitamin C to a solution containing only iron LF was found to have a strong astringent taste, contain free iron (100-fold or more), and form precipitates.

**[Table 1]**

| Sample | Appearance | Astringent taste | Free iron content |
|---|---|---|---|
| (1) Iron LF composition solution containing skim milk + vitamin C | Have no precipitates | 0 point | 30.0 *µ*g / 100 ml |
| (2) Solution containing only iron LF + vitamin C | Have precipitates | 3 points | 5,400 *µ*g / 100 ml |

### Example 2

An iron LF solution with an iron content of 1% (2 g) and skim milk powder (2 g) were dissolved in deionized water (96 g), and the solution was mixed with stirring using an ultra-disperser (ULTRA-TURRAX T-25, manufactured by IKA Japan K.K.) at 8, 000 rpm for 5 minutes, to thereby yield 100 g of an iron composition solution containing reduced skim milk.

### Test Example 2

To 100 g of the iron composition solution containing reduced skim milk and obtained in Example 2 was added 80 mg of vitamin C, followed by heat sterilization at 85°C for 30 minutes. Sensory evaluation of the solution was performed, and the solution was found to have little astringent taste of iron.

### Example 3

An iron LF solution with an iron content of 1% (40 g), a whey protein isolate (WPI, 76 g), and deionized water (1,884 g) were mixed, and the solution was mixed with stirring using a TK homomixer MARK II (manufactured by PRIMIX Corporation) at 5,000 rpm for 5 minutes and then concentrated four- fold using an RO membrane, to thereby yield 500 g of an iron composition solution containing WPI.

### Test Example 3

To 100 g of the iron composition solution containing WPI and obtained in Example 3 was added 100 mg of vitamin C, and the solution was mixed with stirring, followed by sensory evaluation of the solution. As a result, the solution was found to have little astringent taste of iron.

### Example 4

Heme iron with an iron content of 2% (10 g) and skim milk powder(38 g) were dissolved in deionized water (952 g), and the solution was mixed with stirring using an ultra-disperser (ULTRA-TURRAX T-25; manufactured by IKA Japan K.K.) at 8, 000 rpm for 3 minutes, to thereby yield 1, 000 g of an iron composition solution containing reduced skim milk.

### Test Example 4

To 100 g of the iron composition solution containing reduced skim milk and obtained in Example 4 was added 100 mg of vitamin C, and the solution was mixed with stirring, followed by sensory evaluation of the solution. As a result, the solution was found to have little astringent taste of iron.

### Example 5

Ammonium iron citrate with an iron content of 19.8% (7 g) was dissolved in raw skim milk (3, 993 g), and the solution was mixed with stirring using a TK homomixer (MARK II, manufactured by PRIMIX Corporation) at 4, 000 rpm for 3 minutes, to thereby yield 4, 000 g of an iron composition solution containing raw skim milk.

### Test Example 5

To 100 g of the iron composition solution containing raw skim milk and obtained in Example 5 was added 10 g of sugar and 50 mg of vitamin C, and the solution was mixed with stirring, followed by sensory evaluation of the solution. As a result, the solution was found to have little astringent taste of iron.

### Example 6

Skim milk powder (300 g), an iron LF solution (100 g), fresh cream (1,483 g), an emulsifier (12 g), and a stabilizer (13 g) were added to water (2,400 g), and the solution was stirred using a TK homomixer (MARK II, manufactured by PRIMIX Corporation) at 10,000 rpm for 3 minutes, to thereby yield an iron composition containing reduced skimmilk and fresh cream of the present invention. Thereafter, 660 g of granulated sugar was mixed therein, and the solution was sterilized at 85°C and subjected to homogenization treatment at 100 kg/cm² and 50 kg/cm². Subsequently, the solution was cooled to 10°C or lower in ice water, and 5 g of vitamin C and 12 g of vanilla flavor dissolved in 15 g of water were added thereto, followed by freezing, to thereby yield 5 kg of ice cream. The ice cream was packed into 50 150-ml containers and frozen at -30°C. Two weeks later, the ice cream was evaluated, and as a result, it was found to have little astringent taste of iron.

### Example 7 (not according to the invention)

Skim milk (1 kg), a whey protein isolate (WPI, 400 g), soybean cake (1.2 kg), soybean oil (400 g), corn oil (200 g), palm oil (2.6 kg), wheat flour (900 g), bran (200 g), a vitamin/mineral mixture (1 kg), and cellulose (280 g) were mixed into 20 g of the iron composition containing skim milk which had been obtained in Example 1, and the solution was heat-sterilized at 121°C for 3 minutes, to thereby yield 8.2 kg of a cat food. The resultant cat food was found to have little astringent taste of iron and have no strange flavor.

### Example 8

Maltitol (160 g), vitamin C (1,400 mg), a flavor (600 mg), and water (1, 398 g) were mixed into 40 g of the iron composition containing skim milk which had been obtained in Example 1, and the solution was heat-sterilized at 90°C for 15 minutes and packed into 100-ml glass bottles, to thereby yield 16 bottles of a drink containing the iron composition of the present invention. The resultant drink was found to form no precipitates and have no strange flavor in all the bottles.

### Example 9 (not according to the invention)

Soybean protein (500 g), fish oil (100 g), perilla oil (300 g), dextrin (1.8 kg), a mineral mixture (600 g), a vitamin mixture (198 g), an emulsifier (200 g), a stabilizer (400 g), and a flavor (8 g) were mixed into 200 g of the iron composition obtained in Example 3, and the solution was packed into 100-ml retort pouches and heat-sterilized using a retort sterilizer (a Class 1 pressure vessel) at 121°C for 6 minutes, to thereby yield 4.3 kg of an enteral nutrition product.

### Industrial Applicability

According to the present invention, it is possible to provide a stable iron composition in the presence of a substance having a property of releasing iron from an iron preparation or reducing the released iron, without causing an astringent taste of iron or forming precipitates or the like. In addition, the present invention can provide foods or drinks, animal feeds, and medicines blended with an iron composition including a milk protein and an iron preparation, which is characterized by being stable in the presence of a substance having a property of releasing iron or reducing the released iron.

## Claims

1. Use of a milk protein in a composition comprising iron and vitamin C to avoid an astringent taste of iron or the formation of precipitates, wherein the milk protein is skim milk or whey protein isolate (WPI).

2. Use according to Claim 1, in which 15 parts or more of the milk protein are used with respect to one part of iron.

3. Use according to any one of Claims 1 or 2, in which the composition comprises a raw material generally used in foods or drinks, animal feeds, and medicines.

## Patentansprüche

1. Verwendung eines Milchproteins in einer Zusammensetzung, die Eisen und Vitamin C umfasst, um einen adstringierenden Geschmack von Eisen oder die Bildung von Präzipitaten zu vermeiden, wobei das Milchprotein entrahmte Milch oder Molkeproteinisolat (WPI) ist.

2. Verwendung nach Anspruch 1, wobei 15 Teile oder mehr des Milchproteins mit Bezug auf ein Teil Eisen benutzt werden.

3. Verwendung nach Anspruch 1 oder 2, wobei die Zusammensetzung ein Rohmaterial umfasst, das im Allgemeinen in Lebensmittel oder Getränken, Tierfutter und Medikamenten benutzt wird.

## Revendications

1. Utilisation d'une protéine de lait dans une composition comprenant du fer et de la vitamine C afin d'éviter un goût astringent du fer ou la formation de précipités, où la protéine de lait est du lait écrémé ou un isolat de protéine de lactosérum (WPI).

2. Utilisation selon la revendication 1, dans laquelle 15 parties ou plus de la protéine de lait sont utilisées par rapport à une partie de fer.

3. Utilisation selon l'une quelconque des revendications 1 ou 2, dans laquelle la composition comprend une matière première généralement utilisée dans les aliments ou les boissons, les aliments pour animaux et les médicaments.
